# FASCICULE DE BREVET EUROPEEN

(11) **EP 0 969 835 B1**
(45) Date de publication et mention de la délivrance du brevet: **04.04.2007**
(21) Numéro de dépôt: 98904238.7
(22) Date de dépôt: 28.01.1998
(51) Int. Cl.: A61K 31/44

(54) **UTILISATION D' ANTAGONISTES DES RECEPTEURS AUX CANNABINOIDES CENTRAUX POUR REGULER L'APPETENCE**
VERWENDUNG VON ANTAGONISTEN DER ZENTRALEN KANNABINOIDREZEPTOREN ZUR REGULIERUNG VON SUCHTERSCHEINUNGEN
USE OF CENTRAL CANNABINOID RECEPTOR ANTAGONISTS FOR REGULATING APPETENCY

(30) Priorité: 28.01.1997 FR 9700870
(43) Date de publication de la demande: 12.01.2000
(73) Titulaire: Sanofi-Aventis, 75013 Paris (FR)
(72) Inventeur: MARUANI, Jeanne, F-34570 Vailhauques (FR); SOUBRIE, Philippe, F-34270 Saint Mathieu de Tréviers (FR)
(74) Mandataire: Gillard, Marie-Louise
(86) Numéro de dépôt international: PCT/FR1998/000154
(87) Numéro de publication internationale: WO 1998/032441

(56) Documents cités:
- EP-A- 0 656 354
- D. SHIRE ET AL.: "Structural features of the central cannabinoid CB1 receptor involved in the binding of the specific CB1 antagonist SR 141716A" J. BIOL. CHEM., vol. 271, no. 12, 1996, pages 6941-6946, XP002044759
- R. PERTWEE ET AL.: "AM630, a competetive cannabinoid receptor antagonist." LIFE SCI., vol. 56, no. 23/24, 1995, pages 1941-1947, XP002044760
- M. RINALDI-CARMONA ET AL.: "Biochemical and pharmacological characterisation of SR141716A, the first potent and selective brain cannabinoid receptor anragonist." LIFE SCI., vol. 56, no. 23/24, 1995, pages 1949-1955, XP002044761
- F. BARTH ET AL.: "Cannabinoids: receptors, endogenous ligans and a newly synthesized antagonist." NIDA RES. MONOGRAPH, vol. 162, 1996, pages 52-53, XP002044762
- M. RINALDI-CARMONA ET AL.: "Characterization and distribution of binding sites for [3H]-SR141716A, a selective brain(CB1) cannabinoid receptor antagonist, in rodent brain." LIFE SCI., vol. 58, no. 15, 1996, pages 1239-1247, XP002044763
- M. RINALDI-CARMONA ET AL.: "SR141716A, a potent and selective antagonist of the brain cannabinoid receptor." FEBS LETTERS, vol. 350, no. 2-3, 1994, pages 240-244, XP002044764 cité dans la demande

## Description

La présente invention concerne une nouvelle utilisation du N- pipéridino-5-(4-chlorophényl)-1-(2,4-dichlorophényl)-4-méthylpyrazole-3-carboxamide, de ses sels pharmaceutiquement acceptables et de leurs solvates, qui sont des antagonistes des récepteurs aux cannabinoïdes centraux dits récepteurs CB1.

Plus particulièrement, l'invention se rapporte à l'utilisation de ces antagonistes des récepteurs CB1 pour la préparation de médicaments utiles pour traiter les troubles des comportements consommatoires vis-à-vis des substances appétissantes choisies parmi les sucres, les carbohydrates et l'alcool.

Le delta-9 tétrahydrocannabinol ou Δ⁹-THC est le principal constituant actif extrait de Cannabis sativa (Tuner, 1985 ; In Marijuana 84 Ed. Harvey, DY, IRL Press, Oxford).

Les effets des cannabinoïdes sont dûs à une intéraction avec des récepteurs spécifiques de haute affinité couplés aux protéines G. Deux types de récepteurs sont actuellement décrits : les récepteurs CB1, présents majoritairement au niveau du système nerveux central (Devane et al., Molecular Pharmacology, 1988, 34, 605-613) et les récepteurs CB2 présents dans le système immunitaire (Nye et al., The Journal of Pharmacology and Experimental Therapeutics, 1985, 234, 784-791 ; Kaminski et al., 1992, Molecular Pharmacology, 42, 736-742 ; Munro et al., Nature, 1993, 365, 61-65). La caractérisation de ces récepteurs a été rendue possible par la mise au point de ligands synthétiques tels que le CP 55,940 (J. Pharmacol. Exp. Ther., 1988, 247, 1046-1051), le WIN 55212-2 (J. Pharmacol. Exp. Ther., 1993, 264, 1352-1363) et, plus récemment, par la découverte d'un antagoniste sélectif des récepteurs CB1 : le SR 141716 A (M. Rinaldi-Carmona et al., FEBS Lett., 1994, 350, 240-244).

Des familles de composés ayant une affinité pour les récepteurs aux cannabinoïdes ont été décrites dans plusieurs brevets ou demandes de brevets, notamment la demande européenne EP-576 357, qui décrit des dérivés du pyrazole, et la demande WO 96/02248 qui décrit notamment des dérivés du benzofurane.

Plus particulièrement, le N-pipéridino-5-(4-chlorophényl)-1-(2,4-dichlorophényl)-4-méthylpyrazole-3-carboxamide, également dénommé SR 141716, de formule : 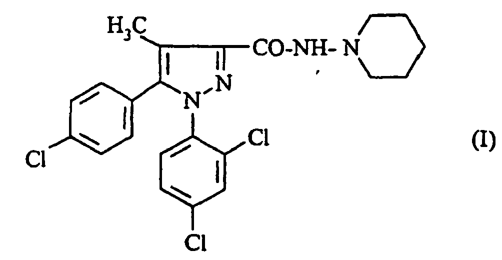 ses sels pharmaceutiquement acceptables et leurs solvates, sont décrits dans la demande de brevet européen EP-656 354, comme antagonistes des récepteurs centraux CB₁.

Le SR 141716 A est le chlorhydrate du SR 141716.

Il est connu que le delta-9 tétrahydrocannabinol dont la dénomination commune internationale est Dronabinol est utilisé pour le traitement de l'anorexie, notamment chez les patients souffrant du SIDA (J. Pain Symptom Manage, 1995, 10 (2), 89-97) ou du cancer (J. Palliat. Care., 1994, 10 (1), 14-18).

De plus il est décrit que le SR 141716 et ses sels qui sont des antagonistes des récepteurs centraux aux cannabinoïdes peuvent être utilisés pour le traitement des troubles de l'appétit, notamment en tant qu'anorexigène, et dans le traitement des troubles liés à l'utilisation de substances psychotropes.

Les anorexigènes classiques provoquent une diminution de l'appétit qui est généralement indépendante des substances alimentaires à consommer.

De façon surprenante, on a maintenant trouvé que le composé de formule (I), ses sels pharmaceutiquement acceptables ou leurs solvates ont une propriété spécifique en agissant de façon élective sur les troubles des comportements consommatoires vis-à-vis des substances appétissantes.

Ainsi, l'administration de ces antagonistes des récepteurs CB1 permet de réguler le désir de consommation pour des éléments non essentiels de l'alimentation tels que les sucres en excès, les carbohydrates en excès ou l'alcool.

En effet, après avoir conduit des essais chez l'animal, on a observé un comportement nouveau de celui-ci : l'animal ne manifeste plus d'appétence spontanée pour l'ingrédient qui lui procure habituellement un plaisir tel que le sucre ou l'alcool, par exemple. Ce manque d'appétence se manifeste également lorsque l'animal a été prétraité par un neuropeptide connu pour augmenter l'appétit tel que le neuropeptide Y (NPY) par exemple.

Pour son utilisation en tant que médicament, le composé de formule (I), ses sels pharmaceutiquement acceptables et leurs solvates doivent être formulés en composition pharmaceutique.

Dans les compositions pharmaceutiques de la présente invention pour l'administration orale, sublinguale, sous-cutanée, intramusculaire, intraveineuse, transdermique, locale ou rectale, le principe actif, peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Dans les compositions pharmaceutiques de la présente invention, le principe actif est généralement formulé en unités de dosage. L'unité de dosage contient de 0,5 à 1000 mg, avantageusement de 1 à 500 mg, de préférence de 2 à 200 mg d'antagoniste des récepteurs CB 1 par unité de dosage pour les administrations quotidiennes.

Lorsque l'on prépare une composition solide sous forme de comprimés, on peut ajouter au principe actif micronisé ou non un agent mouillant tel que le laurylsulfate de sodium et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale, par exemple du beurré de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant, par exemple un alcool tel que l'éthanol ou un glycol tel que le polyéthylèneglycol ou le propylèneglycol, et un tensioactif hydrophile tel que le Tween^{®} 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminée ou à réservoir dans lequel le principe actif est en solution alcoolique.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ- cyclodextrine, 2-hydroxypropyl-β-cyclodextrine ou méthyl-β-cyclodextrine.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

ESSAI N° 1: Effet du SR 141716 A sur la prise d'une solution de sucrose chez le rat.

L'expérience est réalisée selon W.C. Lynch et al., Physiol. Behav., 1993, 54, 877-880.

Des rats mâles Sprague-Dawley pesant 190 à 210 g sont dans un cycle lumineux normal (de 7 heures du matin à 19 heures) et reçoivent de l'eau et de la nourriture ad libitum.

Pendant 6 jours, entre 11 heures et 15 heures, la nourriture et les biberons d'eau sont enlevés et ils sont habitués à boire une solution de sucrose à 5 %.

Les rats buvant moins de 3 g de solution de sucrose sont éliminés.

Le septième jour on réalise l'essai en procédant de la façon suivante :
9 heures : retrait de la nourriture,
10 heures : administration du SR 141716 A par voie orale,
11 heures = T0 : mise en place de biberons contenant une solution pesée de sucrose,
   T0 + 1 heure, T0 + 2 heures, T0 + 3 heures, T0 + 4 heures :
   mesure de la consommation de sucrose par pesée des biberons

**TABLEAU 1**

| Traitement | Nombre de | Consommation solution sucrose en g | | | |
|---|---|---|---|---|---|
| p.o. | rats | 1 Heure | 2 Heures | 3 Heures | 4 Heures |
| Véhicule | 8 | 11,33 | 17,74 | 22,50 | 28,34 |
| 2 ml/kg | | ± 2,50 | ± 4,00 | ± 4.83 | ± 5,01 |
| SR 141716 A | 6 | 5,18 | 9,18 | 12,49 | 16,10 |
| 0,3 mg/kg | | ± 1,61 | ± 2,12 | ± 4,47 | ± 3,95 |
| SR 141716 A | 6 | 3,27* | 3,61** | 5,65* | 7,43** |
| 1 mg/kg | | ± 1,40 | ± 1,40 | ± 2,23 | ± 2,81 |
| SR 141716 A | 6 | 2,95* | 5,41* | 6,96* | 8,58** |
| 3 mg/kg | | ± 1.20 | ± 1,33 | ± 2,15 | ± 2.92 |

| | | | | | |
|---|---|---|---|---|---|
| * p < 0,05 ; ** p < 0,01, test de Dunnett. | | | | | |

D'après les résultats reportés dans le TABLEAU 1, on constate que l'administration du SR 141716 A réduit très nettement la consommation d'eau sucrée, dès la dose de 0,3 mg/kg.

ESSAI N° 2 : Effet du SR 141716 A sur la consommation d'une solution alcoolique chez la souris.

Des souris mâles C 57 BL 6 (Iffa-Credo) sont isolées le jour de leur arrivée dans une animalerie en cycle inversé (nuit de 10 heures à 22 heures) avec 2 biberons remplis d'eau. Après 1 semaine, l'un des biberons d'eau est remplacé par un biberon rempli d'une solution d'alcool à 10 % pendant 6 heures de test. Chaque jour, 30 minutes avant la mise en contact avec le biberon d'alcool, les souris sont traitées par voie sous-cutanée avec du SR 141716 A. Les quantités d'alcool et d'eau bues sont mesurées au bout de 6 heures. Le test est répété pendant 4 jours.

**TABLEAU 2**

| Traitement mg/kg/sc SR 141716 A | Nombre de souris | Quantité d'alcool bue en g à J4 | Quantité d'eau bue en g |
|---|---|---|---|
| Véhicule | 20 | 1,9 ± 0,1 | 1.1 ± 0.1 |
| 0,1 | 10 | 1.4 ± 0.2 | 1.1 ± 0.3 |
| 0.3 | 10 | 1.3 ± 0,2 | 1,1 ± 0,3 |
| 1 | 10 | 1,1 ± 0,2** | 1,3 ± 0.1 |
| 3 | 10 | 1,0 ± 0,2** | 1,6 ± 0.3 |

| | | | |
|---|---|---|---|
| ** p < 0,01, test de Dunnett. | | | |

Les résultats montrent que pour les animaux traités, la consommation d'alcool diminue de façon très sensible : de 1,9 ± 0,1 g pour un animal non traité à 1,0 ± 0,2 g pour un animal ayant reçu 3 mg/kg de SR 141716 A ; parallèlement, la consommation d'eau a augmenté : de 1,1 ± 0,1 à 1,6 ± 0,3 g.

### EXEMPLE 1 Gélule dosée à 1 mg d'antagoniste des récepteurs CB1.

| | |
|---|---|
| SR 141716 micronisé | 1,00 mg |
| Amidon de maïs | 51,00 mg |
| Lactose monohydrate | 103,33 mg |
| Polyvidone | 4,30 mg |
| Laurylsulfate de sodium | 0,17 mg |
| Carboxyméthyl cellulose de sodium réticulée | 8,50 mg |
| Eau purifiée: Q.S. pour granulation humide | |
| Stéarate de magnésium | 1,70 mg |

Pour une gélule blanc opaque n° 3 remplie à 170 mg

### EXEMPLE 2 Gélule dosée à 10 mg d'antagoniste des récepteurs CB1.

| | |
|---|---|
| SR 141716 A micronisé | 10,00 mg |
| Amidon de mais | 51,00 mg |
| Lactose monohydrate | 94,33 mg |
| Polyvidone | 4,30 mg |
| Laurylsulfate de sodium | 0,17 mg |
| Carboxyméthylcellulose de sodium réticulée | 8,50 mg |
| Eau purifiée : Q.S. pour granulation humide | |
| Stéarate de magnésium | 1,70 mg |

Pour une gélule blanc opaque n° 3 remplie à 170 mg

### EXEMPLE 3 Gélule dosée à 30 mg d'antagoniste des récepteurs CB1.

| | |
|---|---|
| SR 141716 micronisé | 30,00 mg |
| Amidon de maïs | 51,00 mg |
| Lactose monohydrate | 74,33 mg |
| Polyvidone | 4,30 mg |
| Laurylsulfate de sodium | 0,17 mg |
| Carboxyméthylcellulose de sodium réticulée | 8,50 mg |
| Eau purifiée : Q.S. pour granulation humide | |
| Stéarate de magnésium | 1,70 mg |

Pour une gélule blanc opaque n° 3 remplie à 170 mg

### EXEMPLE 4 Comprimé dosé à 30 mg d'antagoniste des récepteurs CB1.

| | |
|---|---|
| SR 141716 micronisé | 30,00 mg |
| Lactose monohydrate | Q.S. |
| Amidon de maïs | 40.00 mg |
| Hydroxypropylméthylcellulose 6cP | 5,00 mg |
| Eau purifiée : Q.S. pour granulation humide | |
| Carboxytnéthylcellulose de sodium réticulée | 10,00 mg |
| Stéarate de magnésium | 2,00 mg |

Pour un comprimé terminé à 200 mg.

## Revendications

1. Utilisation du N-pipéridino-5-(4-chlorophényl)-1-(2,4-dichlorophényl)-4-méthylpyrazole-3-carboxamide, d'un de ses sels pharmaceutiquement acceptables ou d'un de leurs solvates pour la préparation de médicaments utiles pour traiter les troubles des comportements consommatoires vis-à-vis des substances appétissantes choisies parmi les sucres, les carbohydrates et l'alcool.

2. Utilisation selon la revendication 1, dans laquelle les substances appétissantes sont les sucres ou les carbohydrates.

3. Utilisation selon la revendication 1, dans laquelle les substances appétissantes sont les sucres.

4. Utilisation selon la revendication 1, dans laquelle les substances appétissantes sont l'alcool.

## Claims

1. Use of N-piperidino-5-(4-chlorophenyl)-1-(2,4-dichlorophenyl)-4-methylpyrazole-3-carboxamide, one of its pharmaceutically acceptable salts or one of their solvates for the preparation of a drug useful for treating the consumption behaviour disorders pertaining to appetizing substances selected among sugars, carbohydrates and alcohol.

2. Use according to claim 1, wherein the appetizing substances are sugars or carbohydrates.

3. Use according to claim 1, wherein the appetizing substances are sugars.

4. Use according to claim 1, wherein the appetizing substances are alcohol.

## Patentansprüche

1. Verwendung des N-Piperidino-5-(4-Chlorphenyl)-1-(2, 4-Dichlorphenyl)-4-Methylpyrazol-3-Carboxamid, eines seiner pharmazeutisch verträglichen Salze oder eines ihrer Solvate zur Herstellung von Medikamenten, die zum Behandeln von Konsumierverhaltensproblemen hinsichtlich schmackhafter Substanzen, die unter Zuckern, Kohlenhydraten und Alkohol gewählt sind.

2. Verwendung nach Anspruch 1, wobei die schmackhaften Substanzen Zucker oder Kohlenhydrate sind.

3. Verwendung nach Anspruch 1, wobei die schmackhaften Substanzen Zucker sind.

4. Verwendung nach Anspruch 1, wobei die schmackhaften Substanzen Alkohol sind.
